Europäisches Patentamt

European Patent Office

Office européen des brevets

(11) Publication number: **0 516 896 A1**

# EUROPEAN PATENT APPLICATION

(21) Application number: **91201937.9**

(22) Date of filing: **24.07.91**

(51) Int. Cl.⁵: **A61K 31/65**

(30) Priority: **03.06.91 IT TO910417**

(43) Date of publication of application:
**09.12.92 Bulletin 92/50**

(84) Designated Contracting States:
**DE ES FR GB**

(71) Applicant: **TEKNOFARMA S.p.A.**
**Strada comunale da Bertolla all'Abbadia di**
**Stura 14**
**I-10156 Torino(IT)**

(72) Inventor: **Colombatti, Vittorina**
**Strada del Salino 37**
**I-10133 Torino(IT)**

(74) Representative: **Spandonari, Carlo**
**corso Re Umberto 56**
**I-10128 Torino(IT)**

(54) Chlortetracycline hydrochloride for use as antiprotozoary preparation for cats and dogs.

(57) The veterinary antiprotozoary preparation is intended for treatment of coccidiosis, particularly due to the genera Isospora and Sarcocystis, and it comprises chlortetracycline hydrochlorate in a neuter excipient, for oral administration. Preferably, chlortetracycline hydrochlorate is administered with a posology of ca. 25 mg pro die per kg of body weight of the subject under treatment, for a duration of 6 to 15 days.

This invention is concerned with a veterinary antiprotozoary preparation for small animals, particularly cats and dogs, and to a process for using the preparation.

A satisfactory treatment has not been available until now for coccidiosis in small house pets, caused by parasites such as various species of Isospora and of Sarcocystis. Disinfectants such as quinacrine and atebrin, etc., have been used in the past, but with only limited results. Sulphonamide treatment is popular nowadays, but it has a limited effectiveness only toward Isospora canis, Isospora rivolta and Isospora felis, while its effectiveness against Isospora bigemina is extremely poor. Moreover, treatment with sulphonamides must be continued for a long time. In any case, neither known chemotherapeutic treatments nor sulphonamides can achieve positive results in 100% of the cases. Another drawback is that coccidia tend to develop resistance to sulphonamides.

It is therefore the main object of the invention to provide a veterinary preparation for treatment of canine and feline coccidiosis supported by the genera Isospora and Sarcocystis.

The invention achieves the above and other objects and advantages, such as will appear from the following disclosure, with a veterinary antiprotozoary preparation for cats and dogs, particularly against infestations by Isospora and Sarcocystis, characterized in that it comprises chlortetracycline hydrochlorate in a neuter excipient, for oral administration.

The invention will now be described in more detail with reference to preferred embodiments.

## PROPERTIES OF TETRACYCLINES

It is known, see e.g. M. A. Sande and G. L. Mandell, Farmaci antibatterici: Tetracicline e Cloramfenicolo, 1264-1274, how to extract chlortetracycline from fermentation of cultures of Streptomyces aureofaciens.

From the above and several other publications, the wide-spectrum bacteriostatic properties of all tetracyclines are known. Such properties substantially depend on two effects, i.e. the block of protein synthesis, which acts at the level of the 50S sub-units in the ribosomes; and the chelating effect of bivalent cations, which inhibits several enzyme systems in the bacteria. Vice versa, the resistance of yeasts and fungus to tetracyclines is also known.

From a pharmacodynamic point of view, tetracyclines are stable in the acid gastric environment as well as in the alkaline intestinal environment, and therefore may be administered by mouth. Although certain tetracyclines are almost completely absorbed (90-95%), chlortetracycline has a moderate absorption, less than 30% by mouth. This is due, on the one hand to a limited solubility in alkaline environment, and on the other hand to its combination with calcium in the food.

Tetracyclines, when absorbed, rapidly spread through the tissues, at both an extra- and an intra-cellular level, and particularly in milk, bones, teeth. The placentary circle is easily crossed (see, e.g., M. H. Pindell, K. M. Cull, K. M. Doran & H. L. Dickinson, Absorption and excretion studies on tetracycline, J. Pharm. Exp. Therap., 1959, 125:287).

Tetracyclines are biotransformed in a relatively low percentage, about 30%, with the exception of chlortetracycline, which, on the contrary, undergoes a substantial biotransformation. As a consequence, while the other tetracyclines are mostly eliminated in a non-metabolized form, chlortetracycline is eliminated, in a large percentage, in a metabolized form, mainly through the kidneys and secondarily in the bile (20-25% of the administered dose). The fraction eliminated in the bile in non-biotransformed form undergoes a reabsorption at enteric level.

Studies are known concerning tetracycline and chlortetracycline in the dog (see W. D. Gray, R. T. Hill, R. Winne & R. W. Cunningham, Aureomycin absorption and distribution in the gastro-intestinal tract of the dog, J. Pharm. Exp. Therap., 1953, 109:223). From such studies it appears that both the above-mentioned antibiotics, after intravenous administration to the dog of 10 mg per kg of weight, penetrate all tissues well, except for fat tissues. Generally, chlortetracycline appears to spread better than tetracycline, except for the teeth, where chlortetracycline has a poorer diffusion.

From the above-mentioned works, however, it appears that, in order to have an effective spread in all tissues, it is necessary, in the case of chlortetracycline, to administer the drug intravenously. Appreciable concentrations will otherwise be found only at intestinal level.

Toxic effects of tetracyclines are extremely low at acute level, although certain secondary effects can be found, e.g. as described in M. Finland, M.E. Grigsby & T.H. Haight, Efficacy and toxicity of oxytetracycline (terramycin) and chlortetracycline (aureomycin), A.M.A. Arch. Int. Med., 1954, 94:23.

The lethal dose (DL 50) of tetracyclines turns out to be about 10,000 mg/kg/die.

## CANINE AND FELINE COCCIDIOSIS

Coccidiosis is a parasitic disease supported by sporozoa of the Order Coccidia, and able to affect both cats and dogs. Pathogenic coccidia for the former belong to the genera Isospora e Sarcocystis. The disease has an incubation of 4 to 7 days, and begins with general symptoms (such as loss of weight, pallor of the mucous membrane, conjunctival catarrh, slight hyperthermia, acute thirst, diarrhea). Systemic symptoms then follow, such as retracted abdomen or volume increase of the abdomen, tenderness under palpation, intestinal meteorism, sometimes vomit. Other ailments affecting the nervous system may appear, particularly in pups.

In very young subjects, the course of coccidiosis can be hyperacute, with early haemorrhagic diarrhea and death in 2 to 3 days. In the pups, the course is usually acute, with duration of 15 to 20 days. In subjects who have overcome the acute form, coccidiosis often persists in a chronic form, with chronic elimination of oocysts and with periodical diarrhea, though mostly with no other apparent symptoms.

THERAPY ACCORDING TO THE INVENTION

For the treatment of coccidiosis in cats and dogs, the invention provides for oral administration of 25 mg/die chlortetracycline hydrochlorate per kg of body weight of the subject, for an average duration of 6 to 15 consecutive days, until disappearance of oocysts in copromicroscopic examinations.

The preparation according to the invention contains, as an active principle, chlortetracycline hydrochlorate. Although use of tetracyclines as antibacterial agents is known, their usefulness as antiprotozoary remedies has not been known or verified. Although a few Authors had previously suggested to use tetracyclines in the therapy of canine coccidiosis, such treatment was believed to be objectionable, due to the strong diffusion of tetracyclines in organic tissues, particularly in bones and teeth, which will impart a permanent, undesired yellowish coloration of the teeth.

The invention is based on the recognition that chlortetracycline, in contrast to other tetracyclines of a fermentative or semisynthetic origin, is poorly absorbed at the intestinal level and does not cause coloration of the teeth at the concentrations required for treatment of coccidiosis (25 mg/die per kg of body weight of the subject), which are considerably lower than those required in antibiotic use. The inventor has thus found that chlortetracycline can be used as a therapeutic agent having a strong effectiveness in the treatment of infestations of cats and dogs by Isospora rivolta, Isospora canis, Isospora felis, Isospora bigemina and Sarcocystis, with a dosage of 25 mg pro die per kg of body weight of the subject, and with durations of the treatment of 6 to 15 days, with no undesirable secondary effects such as yellowish coloration of the teeth, notoriously associated with tetracycline treatment.

The absence of yellowish coloration of the teeth is an essential feature among the advantages of the invention, since such coloration would render any remedy unacceptable in the veterinary therapy on cats and dogs, irrespective of the level of efficacy.

In practical use, for an easier dosage in administration, the invention provides a preparation of tablets with the following composition:

| chlortetracycline hydrochlorate: | 100 mg; |
|---|---|
| starch: | 45 mg; |
| lactose: | 25 mg. |

Such tablets will henceforward be designated as "Isospen tablets".

Further, for an easier administration with foods, the invention also provides a preparation of a paste having the following composition:

| chlortetracycline hydrochlorate: | 12.5 g; |
|---|---|
| vaseline: | 70 g; |
| cacao-butter: | 17.5 g. |

Such paste will henceforward be designated as "Isospen paste". For convenience, the paste is preferably packaged in tubes squeezable through a gauged bore.

TESTS OF TOLERABILITY

Experiments were conducted to test the level of fixation of chlortetracycline in the subjects' teeth and the consequent coloration.

Treatments with chlortetracycline by mouth were made on four crossbred dog pups of medium size. Treatment was started at the ages of 40, 60, 70, 80, 100 e 120 days, respectively, with a dosage of 25 mg/kg pro die, for 15 consecutive days. When the pups reached the age of $8\frac{1}{2}$ months, the adult dentition being complete, the pups' oral cavities were inspected at normal and at ultraviolet light. The pups were then sacrificed in order to determine any residues of chlortetracycline in their teeth. Accordingly, the teeth were reduced to a micronized powder, and determinations were made according to the microbiological and the HLPC methods, in order to produce evidence of any existing chlortetracycline.

The results of the examinations were as follows.

Inspection of oral cavity

No morphological alteration of the teeth was shown.

Microbiological method

To 100 mg powder, 5 ml of 0.1N HCl were added, while stirring in a mixer for 1 hour, and subsequently collecting 5 ml of float. These were used for the microbiological determination, by using Bacillus cereus ATCC 9634, the method having a sensitivity of 0.2 mcg/ml. The examination had a negative outcome.

HPLC method

To 500 mg powder, 5 ml of 0.1N HCl were added, while stirring in a mixer for 2 to 4 hours, and subsequently collecting the float for determination.

For the chromatographic analysis, a liquid chromatograph Perkin Elmer Series 400 was used, coupled with a detector LC235. Data acquisition and chromatographic trace were obtained by means of Laboratory Computing Integrator LC100 of Perkin Elmer.

The following combination was used:

stationary phase: column Merck C8/100 Licrosphere - granulometry 5 $\mu$m 250x4 mm i.d.

mobile phase: elution water:$CH_3CN$ = 18:82 in isocratic conditions. The elution water was prepared as follows: 0.170 g of $KH_2PO_4$ were dissolved in 500 ml of water for HPLC with 0.3% phosphoric acid, and 0.3 ml of 40% solution of tetrabutylammonium hydroxide were added.

The flow was 1.5 ml/min. The system was thermostatized at 35 °C. Detection of the elution peak was made at 265 nm (tr = 4.0 min). Identification and determination of the purity of the active substance in the sample under analysis was made by comparison with the retention time and the UV spectrum of the standard. Quantitative determinations were obtained by the method of the external standard. The injected volume was 20 $\mu$litres in each case.

The standard solution was 100 mg chlortetracycline hydrochlorate (USP) in 100 ml in 0.01N HCl. 5 ml of this solution were diluted to 100 ml with the mobile phase. In the chromatograph were injected 20 $\mu$litres of such standard solution for the calibration steps.

A calibration curve of concentration vs. area was constructed with the help of stepped standard solutions. The analytical method proposed turns out to be linear in the concentration range of chlortetracycline hydrochlorate 0.004 to 0.15 mg/ml. Sensitivity is 10 nanograms; reproducibility of the method is within ± 2%.

Determination by the HPLC method also gave a negative result.

TESTS OF EFFICACY IN DOGS

Several clinical cases are described below, which show the effectiveness of the therapy of coccidiosis in dogs by means of chlortetracycline.

Example 1

Chlortetracycline was administered to a male cross of 30 kg body weight, infested by Isospora rivolta, in the posology of 20 mg/kg pro die. After 15 days therapy, the search for occysts in the feces was negative.

Example 2

Isospen paste was administered to a male crossbred pup of 1 kg body weight, infested by Isospora canis and Isospora rivolta, in the equivalent dosage of 20 mg/kg chlortetracycline pro die. After 11 days therapy, the search for oocysts in the feces was negative.

Example 3

A male cross of 7 kg body weight, infested by I. canis, was treated with Isospen tablets in the equivalent dosage of 20 mg/kg chlortetracycline pro die. After 15 days therapy, the search for oocysts in the feces was positive.

Example 4

A female cross of 7 kg body weight, infested by I. canis and I. rivolta, was treated with chlortetracycline in the dosage of 20 mg/kg pro die. After 9 days therapy, the search for oocysts in the feces was positive.

Example 5

A male crossbred pup of 4 kg body weight, infested by I. canis and I. rivolta, was treated with Isospen paste in the equivalent dosage of 25 mg/kg chlortetracycline pro die. After 6 days therapy, the search for oocysts in the feces was negative.

Example 6

A male hound of 10 kg body weight, infested by I. canis and I. rivolta, was treated with Isospen tablets in the equivalent dosage of 20 mg/kg chlortetracycline pro die. After 15 days therapy, the search for oocysts in the feces was negative.

Example 7

A female cocker of 14 kg body weight, infested by I. rivolta, was treated with Isospen tablets in the equivalent dosage of 25 mg/kg chlortetracycline pro die. After 3 days the search for oocysts in the feces was still positive; after 5, 6 and 7 days a progressive decrement was noticed, as well as the appearance of oocysts having morphological alterations; from the 11th day, the searches always resulted negative, until the 15th day. Treatment was interrupted after 12 days.

Example 8

A female German shepherd of 25 kg body weight, with pups, infested by I. rivolta, was treated with Isospen tablets in the equivalent dosage of 25 mg/kg pro die. After 5, 6, 7, 8 and 9 days, copromicroscopic searches for oocysts were negative. Treatment was suspended after 7 days.

Example 9

A male collie of 21 kg body weight, infested by I. rivolta, was treated with Isospen paste in the equivalent dosage of 25 mg/kg pro die. Searches of the feces made after 2, 5, 8 and 10 days showed oocysts having morphological alterations, in progressively lower counts; searches made after 12 and 15 days were negative. Treatment was suspended after 13 days.

Example 10

Two male crossbred pups of 2 kg body weight, infested by I. canis, were treated with Isospen paste in the equivalent dosage of 25 mg/kg pro die. Searches of the feces made after 8, 9 and 10 days gave a negative result. Treatment was suspended after 8 days.

Example 11

An adult male boxer of 27 kg body weight, infested by Sarcocystis, was treated with Isospen tablets in the equivalent dosage of 25 mg/kg chlortetracycline pro die, for 10 days. At the end of the treatment, copromicroscopic searches for oocysts were negative.

Example 12

Two adult male crosses of ca. 20 kg body weight, infested by Sarcocystis, were treated with Isospen tablets in the equivalent dosage of 25 mg/kg chlortetracycline pro die, for 10 days. At the end of the treatment, copromicroscopic searches for oocysts were negative.

Example 13

A male cross of 12 kg weight, infested by I. canis and I. rivolta, was treated with sulphamethoxine intramuscularly, at a dosage of 20 mg/kg pro die, for 8 consecutive days. A copromicroscopic search for oocysts made after 8 days was still positive. Treatment with Isospen tablets followed, at an equivalent dosage of 25 mg/kg chlortetracycline pro die. After 8, 9 and 11 days of treatment, search in the feces showed oocysts having morphological alterations and receding. After 15 days, the search was negative.

Example 14

A male cross of 18 kg weight, infested by I. canis and I. rivolta, was treated intramuscularly with sulphamonomethoxine, at a dosage of 20 mg/kg pro die, for 6 consecutive days. A copromicroscopic search for oocysts made after 6 days was positive for the presence of numerous oocysts. A treatment with Isospen paste followed, at the equivalent dosage of 25 mg/kg chlortetracycline pro die. After 7, 9 and 15 days treatment, the search for oocysts in the feces was negative.

Example 15

A male cross of 4.3 kg body weight, infested by Isospora canis, was treated with mepacrine by mouth at a dosage of 10 mg/kg pro die. After 17 days there was a clinical improvement, but a copromicroscopic search for oocysts was still positive.

Example 16

A male cross of 7 kg body weight, infested by Isospora canis, was treated with sulphadimethoxine by mouth at a dosage of 50 mg/kg pro die. After 13 days a copromicroscopic search for oocysts was still positive. A treatment followed with Isospen paste at an equivalent dosage of 25 mg/kg chlortetracycline pro die. After 12 days treatment, the search in the feces was negative.

TESTS OF EFFICACY IN CATS

Several clinical cases are described below, which show the effectiveness of the therapy of coccidiosis in cats by means of chlortetracycline.

Example 17

A male European cat of 4 months' age, infested by Isospora felis, was treated with Isospen pasta in the equivalent dosage of 25 mg/kg chlortetracycline pro die for 12 days. At the end of the treatment, the copromicroscopic search was negative.

Example 18

An adult female European cat, infested by Isospora felis, was treated with Isospen pasta in the equivalent dosage of 25 mg/kg chlortetracycline pro die for 14 days. At the end of the treatment, the copromicroscopic search for oocysts was negative.

Example 19

An adult male European cat, infested by Isospora felis, was treated with Isospen pasta in the equivalent dosage of 25 mg/kg chlortetracycline pro die. After 15 days, the copromicroscopic search for oocysts was negative.

Example 20

Four whelps of European cat, infested by Isospora felis, were treated with Isospen pasta in the equivalent dosage of 25 mg/kg chlortetracycline pro die. After 12 days, the copromicroscopic search for oocysts was negative.

CONCLUSIONS

From the above disclosed examples of clinical cases in cats and dogs, as well as from field experience, it can be concluded that chlortetracycline, when administered by mouth with a posology of 25 mg pro die per kg of body weight of the subject, has an anticoccidic activity substantially stronger than the one of known drugs. In all treated cases, a clear clinical improvement could be seen after only 2 to 3 days, with remission of diarrhea and of the other typical symptoms of coccidiosis. Negativization of copromicroscopic searches took place, depending on circumstances, after 8, 10 or 15 days.

Chlortetracycline hydrochlorate administered by mouth is well tolerated, due, among other things, to the limited absorption at the intestinal level. Concerning dentition, observations have not shown, in the pups treated, any appearance of dental anomalies which could be traced to the treatment performed with the posology and in the manner disclosed above and falling within the teachings of the invention.

**Claims**

1. A veterinary antiprotozoary preparation for cats and dogs, particularly against infestations by Isospora and Sarcocystis, characterized in that it comprises chlortetracycline hydrochlorate in a neuter excipient, for oral administration.

2. The veterinary antiprotozoary preparation of claim 1, against infestations by Isospora rivolta, Isospora bigemina, Isospora canis and Isospora felis.

3. The veterinary antiprotozoary preparation of claim 1 or 2, characterized in that it comprises tablets having the following composition, for each tablet:

| chlortetracycline hydrochlorate: | 100 mg; |
|---|---|
| starch: | 45 mg; |
| lactose: | 25 mg. |

4. The veterinary antiprotozoary preparation of claim 1 or 2, characterized in that it comprises a paste having the following composition, for each 100 grammes:

| chlortetracycline hydrochlorate: | 12,5 g; |
|---|---|
| vaseline: | 70 g; |
| cacao-butter: | 17,5 g. |

5. A therapeutic process for coccidiosis in cats and dogs, as caused by Isospora rivolta, Isospora bigemina, Isospora canis or Isospora felis, characterized in that chlortetracycline hydrochlorate is administered by mouth with a posology of less than 60 mg pro die per kg of body weight of the subject under treatment, during not more than 20 days.

6. The therapeutic process of claim 5, characterized in that chlortetracycline hydrochlorate is administered by mouth with a posology of ca. 25 mg pro die per kg of body weight of the subject under treatment, for a duration of 6 to 15 days.

7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | AMERICAN JOURNAL OF VETERINARY RESEARCH, vol. 37, no. 6, June 1976, pages 657-660; O.A. ONAWUNMI et al.: "Suppression and control of experimentally induced porcine coccidiosis with chlortetracycline combination, buquinolate, and lincomycin hydrochloride" --- | | A 61 K  31/65 |
| A | AMERICAN JOURNAL OF VETERINARY RESEARCH, vol. 36, no. 4, part 2, April 1975, pages 593-596; W.M. REID: "Progress in the control of coccidiosis with anticoccidials and planned immunization" ----- | | |

| | |
|---|---|
| | TECHNICAL FIELDS SEARCHED (Int. Cl.5) |
| | A 61 K |

The present search report has been drawn up for all claims

| Place of search | Date of completion of the search | Examiner |
|---|---|---|
| THE HAGUE | 22-01-1992 | KLAVER T. |